(19) Europäisches Patentamt
European Patent Office
Office européen des brevets

(11) Veröffentlichungsnummer: **0 326 922**
**A2**

(12) # EUROPÄISCHE PATENTANMELDUNG

(21) Anmeldenummer: **89101257.7**

(22) Anmeldetag: **25.01.89**

(51) Int. Cl.[4]: **C12P 1/04 , C12P 7/44 , C07G 11/00 , A61K 35/66 , C12N 1/20 , //(C12P1/04, C12R1:01),(C12N1/20, C12R1:01)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4305

(30) Priorität: **30.01.88 DE 3802744**

(43) Veröffentlichungstag der Anmeldung: **09.08.89 Patentblatt 89/32**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Roy, Kirity, Dr. G-31, Hoechst Quarters**
**Darga Road Mulund (West)**
**Bombay 400 082(IN)**
Erfinder: **Chatterjee, Sugata, Dr. H1/2 Hoechst Quarters**
**Darga Road Mulund (West)**
**Bombay 400 082(IN)**
Erfinder: **Vijayakumar, Erra, Koteswara Sayta, Dr.**
**K-3, Hoechst Quarters Darga Road Mulund (West)**
**Bombay 400 082(IN)**
Erfinder: **Rupp, Richard Helmut, Dr.**
**Roederweg 16a**
**D-6240 Königstein/Taunus(DE)**
Erfinder: **Ganguli, Bimal Naresh, Dr.**
**"Saurayuth" 173 Central Avenue**
**Chembur Bombay 400 071(IN)**

(54) **Ein neues pilztötendes Antibiotikum, Isobongkrekinsäure, ein mikrobiologisches Verfahren zu seiner Herstellung und seine Verwendung als Arzneimittel.**

(57) Isobongkrekinsäure, eine Verbindung der Formel

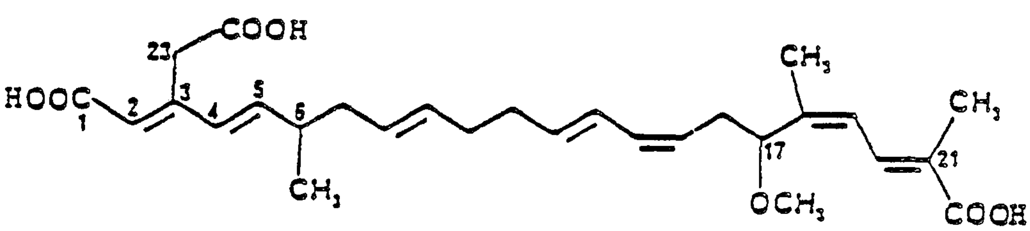

mit pilztötender antibiotischer Wirkung, wird mittels des Eubakterien-Stammes Y-84,0700 (DSM 4305) gewonnen.

EP 0 326 922 A2

## Ein neues pilztötendes Antibiotikum, Isobongkrekinsäure, ein mikrobiologisches Verfahren zu seiner Herstellung und seine Verwendung als Arzneimittel

Die vorliegende Erfindung betrifft ein neues, als Isobongkrekinsäure bezeichnetes pilztötendes Antibiotikum sowie ein Verfahren zu dessen Herstellung aus der Bakterienkultur Y-84,0700 (hinterlegt bei der deutschen Sammlung für Mikroorganismen am 17.11.1987 unter der Nr. DSM 4305) und dessen Mutanten und Varianten.

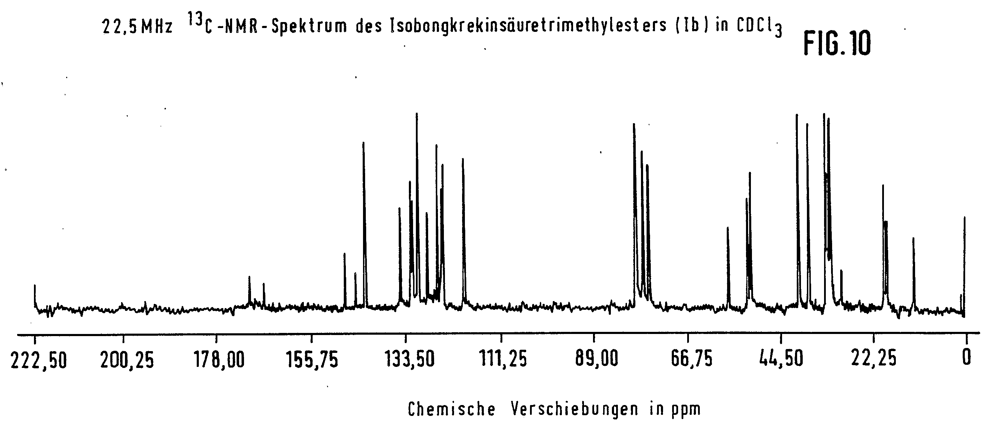

Isobongkrekinsäure (I ) läßt sich als Derivat einer langkettigen Fettsäure beschreiben. Isobongkrekinsäure ließe sich chemisch als 3-Carboxymethyl-17-methoxy-6,18,21-trimethyldocosa-2E,4E,8E,12E,14Z,18Z,20E-hepta-en-1,22-disäure der in Figur 1 gezeigten Formel beschreiben. Ein anderes in der Literatur beschriebenes Antibiotikum dieser Gruppe, das entsprechende Δ 2,3-Z-Isomer, ist Bongkrekinsäure.

Bongkrekinsäure ist in Tetrahedron, 26, 5993, (1970), Tetrahedron, 27, 1839 (1971), Tetrahedron, 29, 1541 (1973) sowie im CRC Handbook of Antibiotic Compounds (Handbuch der Antibiotika), Band VI, S. 393-401 im Abschnitt "Fettsäurederivate" beschrieben. Die Bongkrekinsäure herstellenden Organismen wurden als Pseudomonas cocovenans beschrieben.

Aus den vorliegenden Daten geht jedoch hervor, daß sich Isobongkrekinsäure deutlich von Bongkrekinsäure und allen anderen bekannten langkettigen Fettsäureantibiotika unterscheidet.

Das für die Gewinnung von Isobongkrekinsäure verwendete Eubakterium, Kultur Nr. Y-84,0700, wurde aus einer bei Poona, Maharashtra, Indien, gesammelten Bodenprobe isoliert und als Eubakterium befunden.

Gegenstand der vorliegenden Erfindung ist weiterhin ein Verfahren zur Gewinnung des neuen Antibiotikums Isobongkrekinsäure. Dieses Verfahren ist dadurch gekennzeichnet, daß man Kultur Nr. Y-84,0700, deren Mutanten und Varianten unter aeroben Bedingungen in einem Kohlenstoff- und Stickstoffquellen sowie anorganische Nährsalze und Spurenelemente enthaltenden Nährmedium züchtet, und jenes Antibiotikum aus der Kulturbrühe isoliert und reinigt.

Bevorzugte Kohlenstoffquellen sind z.B. Glukose, Saccharose, Stärke oder Dextrin. Glukose ist die besonders bevorzugte Kohlenstoffquelle. Als Stickstoffquellen werden bevorzugt Sojabohnenmehl, Trypton, Hefeextrakt, Rindfleischextrakt, Malzextrakt, Maisquellwasser, Pepton oder anorganische Stoffe wie Ammoniumsalze verwendet. Die besonders bevorzugt verwendeten Stickstoffquellen sind Malzextrakt oder eine Kombination aus Malzextrakt und einer oder mehrerer anderer Stickstoffquellen. Anorganische Nährsalze können z. B. Natriumchlorid, Kaliumhydrogen/dihydrogenphosphate oder Calciumcarbonat sein. Als Spurenelemente kommen z.B. Eisen-, Mangan-, Kupfer-, Zink , Kobaltsalze oder Salze anderer Schwermetalle in Betracht.

Die Kultur Nr. Y-84,0700 wird vorzugweise bei Temperaturen zwischen ca. 24°C und 30°C und bei einem pH zwischen ca. 6,0 und 8,0 gezüchtet. Besonders bevorzugt ist seine Temperatur von ca. 26°C (± 1°C) und pH ca. 6,5.

Es ist zweckmäßig, die Fermentation nach ca. 66 bis 96 Stunden abzubrechen, wonach man die höchste Ausbeute des erfindungsgemäßen Antibiotikums erhält. Die Fermentation wird vorzugsweise als Submersfermentation in Schüttelkolben sowie in Laborfermentern über ca. 90 Stunden durchgeführt.

Dem Fermenter kann gegebenenfalls ein Schaumverhütungsmittel zugefügt werden (z.B. Desmophen®, Polyole, Bayer AG, Leverkusen). Der Fermentationsverlauf und die Bildung der erfindungsgemäßen Isobongkrekinsäure können mit Hilfe des bekannten Agarplattendiffusionstest durch Messung der antibakteriellen Wirksamkeit der Kulturbouillon gegenüber Staph. aureus 209 P, Aspergillus niger und Trichophyton mentagrophytes verfolgt werden.

In der dabei entstehenden Kulturbrühe befindet sich Isobongkrekinsäure nur im Kulturfiltrat, welches z.B. durch Zentrifugieren vom Myzel getrennt wird. Isobongkrekinsäure kann mittels einer oder mehrerer bekannter Methoden wie Chromatographie auf polymeren Adsorptionsmaterial, wie z.B. auf dem Produkt Diaion HP-20® (Mitsubishi Chemical Industries, Japan) oder Amberlit XAD-2® (Rohm und Haas Co., USA) oder durch Extraktion mit nicht mit Wasser mischbaren Lösungsmitteln wie z.B. Essigester oder Chloroform oder durch Anionenaustauschchromatographie, z.B. über Austauscher mit Polystyrol-Polyamin-Funktionalität (wie z.B. Amberlit IRA 68 $Cl^-$®) bei vorzugsweise basischem pH aus dem Kulturfiltrat gewonnen werden. Die bevorzugt verwendete Methode ist Adsorption auf Diaion HP-20, gefolgt von Desorption der Verbindung unter Verwendung von geeigneten organischen Lösungsmitteln wie z.B. Methanol, Acetonitril oder wäßrigen Kombinationen dieser Lösungsmittel. Das für die Elution von Isobongkrekinsäure bevorzugt verwendete Lösungsmittel ist $MeOH/H_2O$ 1:1. Durch Entfernen des Lösungsmittels aus den aktiven Eluaten erhält man rohe Isobongkrekinsäure. Diese kann durch Chromatographie auf Substanzen wie z.B. Kieselgel, modifiziertem Kieselgel, Magnesiumsilikatgel, wie z.B. Florisil® (Floridin Co., Va. USA), Zellulose oder lipophilem

Gelfiltrationsmaterial wie z.B. Sephadex LH-20® (Pharmacia Fine Chemicals AB, Schweden) weiter gereinigt werden. Die dabei bevorzugt verwendete Methode ist die des Chromatographierens der rohen Isobongkrekinsäure auf Kieselgel unter Verwendung von $CHCl_3$-MeOH-Mischungen zur Elution, wobei die MeOH-Konzentration auf jeder Stufe schrittweise um ca. 10 % erhöht werden sollte. Die die Isobongkrekinsäure enthaltenden Eluate (Messung durch Bioassay) werden konzentriert und z.B. mit Petroläther, Siedepunkt 40 bis 60°C, angerieben, wodurch man die halbreine Verbindung gewinnt. Die so gewonnene halbreine Isobongkrekinsäure kann durch mehrfaches Chromatographieren auf modifiziertem Kieselgel wie z.B. auf dem Handelsprodukt LiChrosorb® RP-18 (Dimethyloctadecylsilyliertes Kieselgel, 50µ, Fa. Merck, Darmstadt) weiter gereinigt werden. Von den für die Elution des LiChrosorb RP-18-Materials in Frage kommenden Lösungsmitteln wie z.B. MeOH, Acetonitril, Aceton oder deren wäßrige Kombinationen werden vorzugsweise MeOH-$H_2O$-Mischungen als Lösungsmittel verwendet, wobei die MeOH-Konzentration schrittweise um ca. 25 % erhöht werden sollte. Das organische Lösungsmittel wird vorzugsweise im Vakuum aus den aktiven Eluaten entfernt und dann zur Entfernung des Wassers gefriergetrocknet, wodurch man Isobongkrekinsäure als weißes Pulver erhält.

Weiterhin betrifft die Erfindung Arzneimittel mit einem Gehalt an Isobongkrekinsäure. Neben dem genannten Wirkstoff können noch weitere Wirkstoffe mit gleicher oder unterschiedlicher Indikation in dem Arzneimittel enthalten sein. Weiterhin können die Arzneimittel Hilfs- und/oder Trägerstoffe enthalten, die mit dem bzw. den Wirkstoffen in eine geeignete Darreichungsform gebracht werden. Die Darreichungsform ist abhängig von der Darreichungsart des Arzneimittels, z.B. äußerliche oder oriale Anwendung, oder Anwendung durch injizieren.

Die anzuwendende Dosierung der erfindungsgemäßen Arzneimittel ist abhängig von verschiedenen Faktoren wie Darreichungsform des Medikaments und Zustand und Gewicht des Patienten und ist gegebenenfalls empirisch zu ermitteln. Die erfindungsgemäßen Arzneimittel eignen sich zur Behandlung verschiedener Krankheiten, besonders geeignet sind sie zur Behandlung von Krankheiten, die durch eine Pilzinfektion verursacht sind oder an denen eine Pilzinfektion beteiligt ist.

Die Isobongkrekinsäure läßt sich leicht zu den entsprechenden Trialkylestern umsetzen, die ebenfalls Gegenstand der vorliegenden Erfindung sind. Die Umsetzung von Carbonsäuren zu Carbonsäureestern ist allgemein bekannt und z.B. im Organikum, VEB Deutscher Verlag der Wissenschaften, 15. Auflage, Berlin 1977, S. 496 ff. beschrieben. Bevorzugt sind die Trialkylester mit 1-4 C-Atomen in der Esterfunktion, besonders bevorzugt ist der Trimethylester der Isobongkrekinsäure. Letzterer läßt sich z.B. herstellen, indem die Isobongkrekinsäure unter Verwendung einer $CH_2N_2$/MeOH-$H_2O$-Diäthyläthermischung, vorzugsweise unter Kühlung, verestert wird. Das Reaktionsprodukt kann z.B. durch chromatographische Methoden gereinigt werden.

Die folgenden Beispiele sollen zur Illustration der vorliegenden Erfindung dienen:

**Beispiel I**

Isolierung der Kultur Y-84,0700 aus Boden

a) Zusammensetzung des Isolierungsnährmediums

| | |
|---|---|
| Na-propionat | 4 g |
| Glycerin | 10 ml |
| $KH_2PO_4$ | 1 g |
| $Na_2HPO_4$ | 4 g |
| $MgSO_4.7H_2O$ | 100 mg |
| $NH_4NO_3$ | 2 g |
| Agar | 15 g |
| Entmineralisiertes Wasser | 1 Liter |
| Chloramphenicol | 50 mg |
| pH | 6,5 |

Am Ende der Herstellung des oben genannten Nährmediums wird Chloramphenicol in 10 ml 95 %-

igem Äthanol gelöst hinzugegeben und mit dem Nährmedium gemischt. Das Nährmedium wird bei einer Temperatur von 121°C 10 Minuten lang sterilisiert. Vor der Sterilisation wird der pH Wert auf 6,5 eingestellt.

**b) Beschickung mit Erde und Isolierung**

0,05 g getrocknete und pulverisierte Erde einer bei Poona gesammelten Bodenprobe wird in die Mitte einer sterilisierten Petrischale (Durchmesser 7,62 cm (6 Zoll)) plaziert und mit einem Tropfen sterilen destillierten Wassers befeuchtet. Das obengenannte Nährmedium wird auf 45°C abgekühlt und in die Petrischale gegossen (ungefähr 80 ml) und gut verrührt. Man läßt die Mischung aus Erde und Nährmedium sich absetzen und inkubiert sie 2 Wochen lang bei 26°C (± 1°C). Die Petrischale wird in regelmäßigen Abständen inspiziert und die Kultur Y-84,0700 aus den wachsenden Mikroorganismen isoliert.

**Beispiel II**

**Züchtung der Kultur Y-84,0700**

**Zusammensetzung des Züchtungsmediums**

Die Kultur Nr. Y-84,0700 wird auf Sabouraud-Glukoseagar der folgenden Zusammensetzung gezüchtet:

| | |
|---|---|
| Glukose | 40 g |
| Pepton | 10 g |
| $Na_2HPO_4$ | 1 g |
| Agar | 15 g |
| Entmineralisiertes Wasser | 1 Liter |
| pH | 6,5 |

Nach sorgfältigem Auflösen der Bestandteile durch Erwärmen wird die Lösung in Reagenzgläser verteilt und dann 20 Minuten lang bei 121°C sterilisiert. Die Reagenzgläser werden abgekühlt und in Schrägstellung bis zur Verfestigung gelagert. Die gewachsene Kultur Nr. Y-84,0700 wird mit Hilfe einer Drahtöse streifenweise auf die Schrägagars aufgebracht, die bei 26°C (± 1°C) inkubiert werden bis ein gutes Wachstum beobachtet wird. Die gut gewachsenen Kulturen werden in einem Kühlschrank gelagert.

**Beispiel III**

**Fermentation der Kultur Y-84,0700 in Schüttelkolben**

**Zusammensetzung des Impfkulturmediums**

| | |
|---|---|
| Lösliche Stärke | 15 g |
| Sojabohnenmehl | 15 g |
| Glukose | 5 g |
| $CaCO_3$ | 2 g |
| NaCl | 5 g |
| Hefeextrakt | 2 g |
| Maisquellwasser | 1 g |
| $ZnSO_4.7H_2O$ | 0,22 mg |
| $CaCl_2$ | 0,55 mg |
| $MnCl_2.4H_2O$ | 0,5 mg |
| $FeSO_4.5H_2O$ | 0,16 mg |
| $oOCL_2.6H_2O$ | 0,16 mg |
| Entmineralisiertes Wasser | 1 Liter |
| pH vor der Sterilisation | 6,5 |

Das obengenannte Impfkulturmedium wird in 100 ml-Portionen auf 500 ml fassende Erlenmeyerkolben mit großen Öffnungen verteilt und bei 121 °C 20 Minuten lang sterilisiert. Die Kolben werden abgekühlt und dann mit einigen Ösen der obengenannten, gut gewachsenen Kultur von Beispiel II beimpft und dann 60 Stunden lang bei 240 U.p.M. und 26 °C (± 1 °C) geschüttelt. Dies ergibt das Impfkulturmedium für die Beimpfung des Herstellungsmediums der folgenden Zusammensetzung:

Zusammensetzung des Herstellungsmediums

| | |
|---|---|
| Glukose | 10 g |
| Malzextrakt | 20 g |
| Pepton | 10 g |
| $Na_2HPO_4$ | 1 g |
| $ZnSO_4.7H_2O$ | 0,22 mg |
| $CaCl_2$ | 0,55 mg |
| $MnCl_2.4H_2O$ | 0,5 mg |
| $FeSO_4.5H_2O$ | 0,16 mg |
| $CoCL_2.6H_2O$ | 0,16 mg |
| Entmineralisiertes Wasser | 1 Liter |
| pH vor Sterilisation | 6,5 |

Das obengenannte Herstellungsmedium wird in 200 ml-Portionen auf 1 l fassende Erlenmeyerkolben verteilt und 20 Minuten lang bei 121 °C sterilisiert. Die Kolben werden abgekühlt und dann mit dem obengenannten Impfkulturmedium beimpft (1 % v/v). Die Fermentation erfolgt auf einem Rotationsschüttler bei 220 U.p.M. und einer Temperatur von 26 °C (± 1 °C) über 90 Stunden.

Die Bildung von Isobongkrekinsäure wird anhand der antibakteriellen Aktivitätsprofile gegen Staph. aureus 209 P, Aspergillus niger und Trichophyton mentagrophytes bestimmt. Nach dem Abernten wurde die Kulturbouillon zentrifugiert und die Isobongkrekinsäure aus dem Kulturfiltrat isoliert und wie im nachfolgenden beschrieben gereinigt.

**Beispiel IV**

**Züchtung der Kultur Nr. Y-84,0700 in Fermentern**

Stufe I: Herstellung der Impfkultur

In Schüttelkolben

Das Impfkulturmedium von Beispiel III (100 ml) wird in 500 ml fassende weithalsige Erlenmeyerkolben gegeben und der pH vor der Sterilisation auf 6,5 eingestellt. Dann wird es in einem Autoklaven 20 Minuten lang bei 121 °C sterilisiert, man läßt abkühlen und beimpft es mit einigen Ösen der gut gewachsenen Kultur von Beispiel II. Die Kolben werden bei 26 °C (± 1 °C) 60 Stunden lang in einem Rotationsschüttler bei 240 U.p.M. inkubiert. Diese gewachsene Kultur wird zur Beimpfung kleiner Fermenter, wie im nachfolgenden beschrieben, verwendet.

Stufe II: Herstellung der Impfkultur

In kleinen Fermentern

10 Liter des Herstellungsmediums (wie in Beispiel III beschrieben) mit einem vor der Sterilisation auf 6,5 eingestellten pH-Wert und 0,03 % (v/v) Desmophen (Bayer AG) als Schaumverhütungsmittel werden in einen 15 l fassenden Fermenter aus rostfreiem Stahl gegeben und in einem Autoklaven 36 Minuten lang bei 121 °C sterilisiert, abgekühlt und mit 10 % (v/v) Impfkultur der oben beschriebenen Stufe I unter aseptischen Bedingungen beimpft. Anschließend wird 24 Stunden unter Berücksichtigung der folgenden Parameter kultiviert.

| | |
|---|---|
| Temperatur | 26 °C (± 1 °C) |
| Schütteln | 180-200 U.p.M. |
| Belüftung | 6 Liter pro Minute |

Die nach 24 Stunden gewachsene Kultur wird zur Beimpfung des Herstellungsmediums der Stufe III verwendet.

**Stufe III: Fermentation**

(a) Kleine Mengen

10 Liter des Herstellungsmediums (wie in Beispiel III beschrieben) mit einem vor der Sterilisation auf 6,5 eingestellten pH-Wert und 0,03 % (v/v) Desmophen (Bayer AG) als Schaumverhütungsmittel werden in einem 15 l fassenden Fermenter aus rostfreiem Stahl 28 Minuten lang in einem Autoklaven bei 121 °C sterilisiert, abgekühlt und unter aseptischen Bedingungen mit 10 % Impfkultur des Wachstums aus dem Schüttelkolben von Stufe I beimpft. Anschließend wird unter Berücksichtigung der folgenden Parameter kultiviert.

| | |
|---|---|
| Temperatur | 26 °C (± 1 °C) |
| Schütteln | 180-200 U.p.M. |
| Belüftung | 6 Liter pro Minute |
| Ernte | nach 90 Stunden |

**(b) Grosse Mengen**

100 Liter des Herstellungsmediums (wie in Beispiel III beschrieben) mit einem vor der Sterilisation auf 6,5 eingestellten pH-Wert und 0,03 % Desmophen (Bayer AG) in einem 150 l fassenden Fermenter oder 250 l des Herstellungsmediums mit 0,03 % Desmophen (Bayer AG) in einem 390 l fassenden Fermenter werden 28 Minuten lang bei 121 °C in situ sterilisiert und mit 10 % der Impfkultur von Stufe II beimpft. Endvolumen des Kulturmediums:

110 l im 150 l-Fermenter.
275 l im 390 l-Fermenter.
Dabei werden die folgenden Parameter berücksichtigt:

| | |
|---|---|
| Temperatur | 26°C (± 1°C) |
| Schütteln | 90 U.p.M. |
| Belüftung für 150 l | 50 Liter pro Minute |
| Belüftung für 390 l | 8 $m^3$/Hr |
| Ernte | nach 90 Stunden. |

Die Bildung von Isobongkrekinsäure wird anhand der antibakteriellen Aktivitätsprofile gegenüber Staph. aureus 209 P, Aspergillus niger und Trichophyton mentagrophytes bestimmt. Die Kulturbouillon wird nach der Ernte zentrifugiert und die Isobongkrekinsäure aus dem Kulturfiltrat isoliert und gereinigt, wie in Beispiel III beschrieben.

**Isolierung und Reinigung der Isobongkrekinsäure**

Ungefähr 250 l der geernteten Bouillon werden durch Zentrifugieren vom Myzel getrennt. Das resultierende Filtrat (pH 7,3) wird durch eine Säule von 8 l Diaion HP-20® geleitet. Die Säule wird zuerst mit entmineralisiertem Wasser (40 l) gewaschen. Die Elution erfolgt mit $MeOH/H_2O$ 1:1 (40 l). Die Eluate, die Isobongkrekinsäure enthalten, werden konzentriert, was unter verringertem Druck erfolgt, um das Methanol zu entfernen. Die resultierende wäßrige Substanz wird lyophilisiert, wodurch man dunkelbraune rohe Isobongkrekinsäure erhält (670 g).

Rohe Isobongkrekinsäure (670 g) wird einer Mitteldruckflüssigkeitschromatographie über Kieselgel (Porengröße 0,062 - 0,037 mm (mesh 230-400), 3 kg) unterzogen. Die Säule wird nacheinander mit $CHCl_3$ - (1,5 l), 5 % MeOH in $CHCl_3$ (10 l) und 10 % MeOH in $CHCl_3$ (18 l) bei einer Durchflußgeschwindigkeit von 150 ml/Minute eluiert. Die Isobongkrekinsäure wird mit 5 % MeOH in $CHCl_3$ eluiert. Die aktiven, die Säure enthaltenden Eluate werden unter verringertem Druck konzentriert, wobei ein dunkelbraunes Öl entsteht (100 g). Diese ölige Substanz wird mit Petroläther angerieben (2 l), der Petroläther wird abgegossen. Der Vorgang wird sieben Mal wiederholt bis nach dem Abgießen des Petroläthers ein fester Rückstand zurückbleibt (16 g). Dieses feste braune Material (16 g) wird dann zweimal hintereinander einer Mitteldruckflüssigkeitschromatographie über LiChrosorb RP-18, einem Reversphasenträgermaterial, unterzogen. In allen Fälle wird die Säule nacheinander mit $MeOH/H_2O$ 50:50 (15 l), $MeOH/H_2O$ 60:40 (10 l) und $MeOH/H_2O$ 70:30 (15 l) bei einer Durchflussgeschwindigkeit von 40 ml/Minute eluiert. 1 l-fraktionen werden gesammelt. Bei der ersten Reinigung mit der Mitteldruckflüssikgeitschromatographie-RP-18-Säule wird die Isobongkrekinsäure mit $MeOH/H_2O$ 70:30 eluiert, während bei der zweiten Reinigung mit der RP-18-Säule $MeOH/H_2O$ 60:40 zur Elution der Säure benutzt wird. Die aktiven Eluate werden unter verringertem Druck eingeengt und lyophilisiert, wobei man ein hellbraunes Pulver erhält (1,8 g). Die so erhaltene Isobongkrekinsäure wird schließlich mit Hilfe einer dritten RP-18-Säule gereinigt.

Die Säure wird mit $MeOH/H_2O$ 50:50 bei einer Durchflussgeschwindigkeit von 2 ml/Minute gereinigt und Fraktionen von etwa 20 ml werden gesammelt. Die aktiven Fraktionen werden vereinigt, unter verringertem Druck konzentriert und lyophilisiert, so daß man 1 g reine Isobongkrekinsäure als weißes Pulver erhält.

Die Reinheit der Isobongkrekinsäure wird mittels HPLC geprüft [4 x 250 mm LiChrosorb RP-18-säule, Laufmittel: $MeOH/H_2O$ 70:30, Durchflussgeschwindigkeit: 1 ml/Minute, Nachweis bei 234 nm, Papiervorschub: 10 mm/Minute]. Die HPLC-Aufzeichnungen zeigt Fig. 2.

Die Isobongkrekinsäure (100 mg) wird unter Verwendung einer $CH_2N_2/MeOH-H_2O$-Diäthyläthermischung bei 0°C verestert. Der Rohester wird mittels präparativer Dünnschichtchromatographie (Kieselgelplatten 20 x 20 cm, 0,5 mm dick, Lösungsmittel zur Entwicklung: 1,5 %-iges MeOH in $CHCl_3$, Lösungsmittel zur Elution: 5 %-iges MeOH in $CHCl_3$) gereinigt. Der Trimethylester (1 b) wird als farbloses Öl gewonnen (59 mg).

Isobongkrekinsäure und ihr Trimethylester haben die folgenden physikalisch-chemischen Eigenschaften:

| | Isobongkrekinsäure | Isobongkrekinsäuretrimethylester |
|---|---|---|
| Aussehen | Weißes amorphes Pulver | Öl |
| Beschaffenheit | sauer | neutral |
| Schmelzpunkt | 190°C (d) | - |
| Löslichkeit | Wäßriges Alkali, schwerlöslich in MeOH und Wasser | $CHCl_3$, $CH_2Cl_2$,MeOH, Aceton, Äthylacetat und Petroläther |
| [α] 20°C | +93,75° (C 0,016; MeOH/$H_2O$ 60:40 | +27,78° (C 1,4; $CHCl_3$) |
| Molekulargewicht | m/z 552 $(m^+3Na-3H)^+$ (FAB) | m/z $M^+$ 528 (EI) |
| Summenformel | $C_{28}H_{38}O_7$ | $C_{31}H_{44}O_7$ |
| UV: $\lambda_{max.}$ nm (Fig. 3 und 4) | 234; 268 (in MeOH/$H_2O$ 6:4); 236; 254 (in MeOH/$H_2O$ 6:4 + 2 Tropfen 1N NaOH | 236, 268 (in MeOH) |
| IR: $\lambda_{max.}$ $cm^{-1}$ (Fig. 5 und 6) | (KBr) 3400-3200 (breit, -OH), 1670 (-C=O aus -COOH) | 1745 (schmal, C=O aus $COOCH_3$) und 1715 (C=O aus ungesättigtem $-COOCH_3$) |
| $^1H$-NMR ($Me_4Si$) (Fig. 7 und 8) | δ (in $D_2O$) 7,26 (d, J=12Hz,1H) 6,92 (d, J=16Hz, 1H) 6,47 (d, J=12Hz, 1H), 6,38 (dd, J= | δ (in $CDCl_3$) 7,50 (d, J=12Hz,1H), 6,35 (d, J=12Hz,1H), 6,26 (dd, J=14,8, 11Hz,1H) 6,09 (d, J=16Hz,1H), |

| | Isobongkrekinsäure | Isobongkrekinsäuretrimethylester |
|---|---|---|
| | 16,11Hz,1H), 6,09 (t, J=11Hz,1H), 5,89 (dd, J=16, 7,5Hz,1H), 5,81 (dt, J=16,7Hz,1H), 5,71 (s,1H), 5,51 (m,2H), 5,30 (dt, J=11,7Hz,1H), 4,57 (t, J=7Hz,1H), 3,25 (s,3H), 3,14 (s,2H), 2,65-1,95 (m,9H), 1,89 (s, 3H), 1,80 (s,3H), 1,01 (d, J=6,5Hz, 3H) | 6,01 (dd, J=16, 7,5Hz, 1H), 5,99 (t, J=11Hz, 1H), 5,88 (s, 1H), 5,67 (dt, J=14,8, 7Hz, 1H), 5,37 (dt, J=8,6Hz, 2H), 5,20 (dt, J=11, 7Hz,1H), 4,34 (t, J= 7Hz,1H), 3,95 (s, 2H) 3,75 (s,3H), 3,70 (s, 3H), 3,67 (s,3H), 3,20 (s,3H), 1,95-2,65 (m,9H), 1,93 (s,3H), 1,83 (s,3H), 1,00 (d, J=6,5Hz,3H) |
| $^{13}C$ NMR (Fig. 9 und 10) | δ (67,5 MHz.$D_2O$, $Me_4Si$), 183,13, 180,83, 179,12, 143,95, 143,80, 143,57, 139,17, 136,11, 134,64, 133,72, 132,16, 131,15, 129,02, 128,41, 128,26, 127,96, 127,38, 81,34, 58,39, 46,06, 42,12, 39,71, 34,81, 34,30, 34,11, 21,62, 20,24, 15,77 | (22,5 MHz, $CDCl_3$,$Me_4Si$) 170,66, 169,04, 176,09, 148,23, 145,63, 143,25, 134,80, 132,20, 131,76, 130,57, 130,35, 128,08, 125,80, 124,94, 124,50, 119,52, 78,45, 56,45, 51,79, 51,14, 39,76, 37,38, 33,26, 32,94, 32,29, 19,39, 18,74, 12,24 |

Die obigen Daten und die in den chemischen Verschiebungen bei $C_{23}$-$H_2$ und $C_4$-H im $^1H$ NMR-Spektrum des Isobongkrekinsäuretrimethylesters beobachteten Unterschiede zu den für den Bongkrekinsäuretrimethylester (IIb) festgestellten geben Aufschluß über die Struktur (Ia) des pilztötenden Antibiotikums Isobongkrekinsäure.

Die biologischen Eigenschaften der Isobongkrekinsäure, ausgedrückt als MHK gegen verschiedene Mikroorganismen, sind nachstehend aufgeführt:

**MHK der Isobongkrekinsäure**

| Lfd. Nr. | Prüforganismus | MHK-Werte in µg/ml |
|---|---|---|
| 1. | Candida albicans | > 125 |
| 2. | Saccharomyces cerevisiae | > 125 |
| 3. | Hefe 5060 | > 125 |
| 4. | Aspergillus niger | 62,5 |
| 5. | Penicillium digitatum 135 | 62,5 |
| 6. | Fusarium culmorum 100 | 7,8 |
| 7. | Trichophyton mentagrophytes | 125 |
| 8. | Cladosporuim resinae | 250 |
| 9. | Botrytis cinerea 16 | 62,5 |
| 10. | Botrytis cinerea 47 | 15,6 |
| 11. | Botrytis cinerea 57 | 31,2 |
| 12. | Alternaria solani 5 | 62,5 |
| 13. | Cercospora beticola 71 | 15,6 |
| 14. | Pyricularia oryzae 154 | 31,2 |
| 15. | Staph. aureus 209 P | 125 |
| 16. | E. coli 9632 | > 125 |

**Ansprüche**

1. Eine Verbindung der Formel

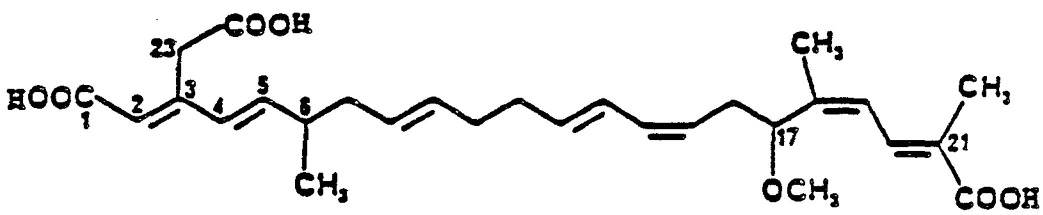

2. Verfahren zur Herstellung der Verbindung gemäß Anspruch 1, dadurch gekennzeichnet, daß man die Bakterienkultur Nr. Y-84,0700 (DSM 4305), deren Mutanten und Varianten unter aeroben Bedingungen auf einem Nährboden, der Kohlenstoff- und Stickstoffquellen, anorganische Nährsalze und Spurenelemente enthält, kultiviert und die Verbindung in üblicher Weise aus der Kulturbrühe isoliert und reinigt.

3. Verfahren nach Anspruch 2, dadurch gekennzeichnet, daß die Kultivierung bei einer Temperatur zwischen 24 °C und 30 °C und bei einem pH zwischen 6 und 8 vorgenommen wird.

4. Verfahren nach Anspruch 2 oder 3, dadurch gekennzeichnet, daß die Kultivierung bei ca. 26 °C (± 1 °C) und pH ca. 6,5 vorgenommen wird.

5. Verfahren nach einem oder mehreren der Ansprüche 2-4, dadurch gekennzeichnet, daß die Kultivierung mindestens ca. 66 Stunden lang durchgeführt wird.

6. Verfahren nach einem oder mehreren der Ansprüche 2-5, dadurch gekennzeichnet, daß die Fermentation als submerse Fermentation durchgeführt wird.

7. Verfahren nach einem oder mehreren der Ansprüche 2-6, dadurch gekennzeichnet, daß die Isobongkrekinsäure durch chromatographische Methoden gereinigt wird.

8. Arzneimittel, gekennzeichnet durch einen Gehalt an der Verbindung gemäß Anspruch 1 neben weiteren Wirkstoffen und/oder Hilfs- und/oder Trägerstoffen.

9. Verwendung der Verbindung gemäß Anspruch 1 zur Herstellung von Arzneimitteln mit pilztötender antibiotischer Wirkung.

10. Eubakterium Y-84,0700 (DSM 4305)

11. Trialkylester der Verbindung gemäß Anspruch 1 mit 1-4 Kohlenstoffatomen in der Esterfunktion.
Patentansprüche für folgenden Vertragsstaat : GR

1. Verfahren zur Herstellung einer Verbindung der Formel I, dadurch gekennzeichnet, daß man die Bakterienkultur

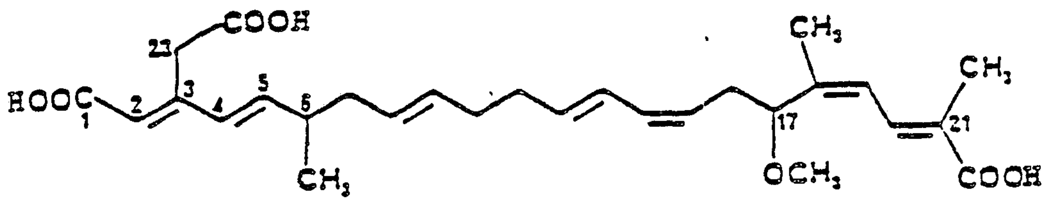

I

Nr. Y-84,0700 (DSM 4305), deren Mutanten und Varianten unter aeroben Bedingungen auf einem Nährboden, der Kohlenstoff- und Stickstoffquellen, anorganische Nährsalze und Spurenelemente enthält kultiviert und die Verbindung in üblicher Weise aus der Kulturbrühe isoliert und reinigt.

2. Verfahren nach Anspruch 1, dadurch gekennzeichnet, daß die Kultivierung bei einer Temperatur zwischen ca. 24° C und 30° C und bei einem pH zwischen ca. 6 und 8 vorgenommen wird.

3. Verfahren nach Anspruch 1 oder 2, dadurch gekennzeichnet, daß die Kultivierung bei ca. 26° C (± 1° C) und pH ca. 6,5 vorgenommen wird.

4. Verfahren nach einem oder mehreren der Ansprüche 1-3, dadurch gekennzeichnet, daß die Kultivierung mindestens ca. 66 Stunden lang durchgeführt wird.

5. Verfahren nach einem oder mehreren der Ansprüche 1-4, dadurch gekennzeichnet, daß die Fermentation als submerse Fermentation durchgeführt wird.

6. Verfahren nach einem oder mehreren der Ansprüche 1-5, dadurch gekennzeichnet, daß die Verbindung der Formel I durch chromatographische Methoden gereinigt wird.

7. Verwendung einer Verbindung, hergestellt gemäß einem oder mehreren der vorausgehenden Ansprüche, zur Herstellung von Arzneimittel mit pilztötender antibiotischer Wirkung.

8. Trialkylester der Verbindung gemäß Anspruch 1 mit 1-4 Kohlenstoffatomen in der Esterfunktion.

9. Verfahren zur Herstellung von Arzneimitteln, dadurch gekennzeichnet, daß man eine Verbindung, die gemäß einem oder mehreren der vorausgehenden Ansprüche hergestellt worden ist, mit weiteren Wirkstoffen und/oder Hilfs- und/oder Trägerstoffen in eine geeignete Darreichungsform bringt.

10. Eubakterium Y-84,0700 (DSM 4305)

Patentansprüche für folgenden Vertragsstaat : ES

1. Verfahren zur Herstellung einer Verbindung der Formel I, dadurch gekennzeichnet, daß man die Bakterienkultur

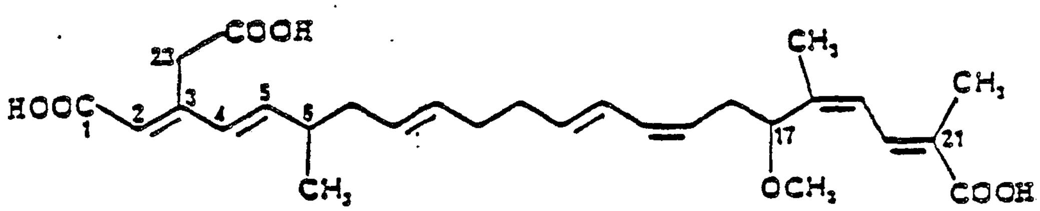

I

Nr. Y-84,0700 (DSM 4305), deren Mutanten und Varianten unter aeroben Bedingungen auf einem Nährboden, der Kohlenstoff- und Stickstoffquellen, anorganische Nährsalze und Spurenelemente enthält kultiviert und die Verbindung in üblicher Weise aus der Kulturbrühe isoliert und reinigt.

2. Verfahren nach Anspruch 1, dadurch gekennzeichnet, daß die Kultivierung bei einer Temperatur zwischen ca. 24° C und 30° C und bei einem pH zwischen ca. 6 und 8 vorgenommen wird.

3. Verfahren nach Anspruch 1 oder 2, dadurch gekennzeichnet, daß die Kultivierung bei ca. 26° C (± 1° C) und pH ca. 6,5 vorgenommen wird.

4. Verfahren nach einem oder mehreren der Ansprüche 1-3, dadurch gekennzeichnet, daß die Kultivierung mindestens ca. 66 Stunden lang durchgeführt wird.

5. Verfahren nach einem oder mehreren der Ansprüche 1-4, dadurch gekennzeichnet, daß die Fermentation als submerse Fermentation durchgeführt wird.

6. Verfahren nach einem oder mehreren der Ansprüche 1-5, dadurch gekennzeichnet, daß die Verbindung der Formel I durch chromatographische Methoden gereinigt wird.

7. Verwendung einer Verbindung, hergestellt gemäß einem oder mehreren der vorausgehenden Ansprüche, zur Herstellung von Arzneimittel mit pilztötender antibiotischer Wirkung.

8. Trialkylester der Verbindung gemäß Anspruch 1 mit 1-4 Kohlenstoffatomen in der Esterfunktion.

9. Verfahren zur Herstellung von Arzneimitteln, dadurch gekennzeichnet, daß man eine Verbindung, die gemäß einem oder mehreren der vorausgehenden Ansprüche hergestellt worden ist, mit weiteren Wirkstoffen und/oder Hilfs- und/oder Trägerstoffen in eine geeignete Darreichungsform bringt.

FIG. 1

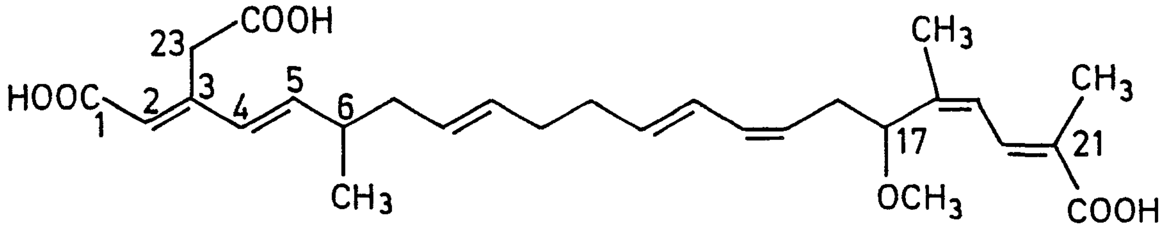

FIG. 2

Kurve der HPLC - Analyse der Isobongkrekinsäure auf einer 4x250mm Lichrosorb RP-18-Säule; Laufmittel: MeOH-$H_2O$ 7:3; Fliessgeschwindigkeit: 1ml / Minute; UV-Nachweis bei A) 268nm, B) 234nm

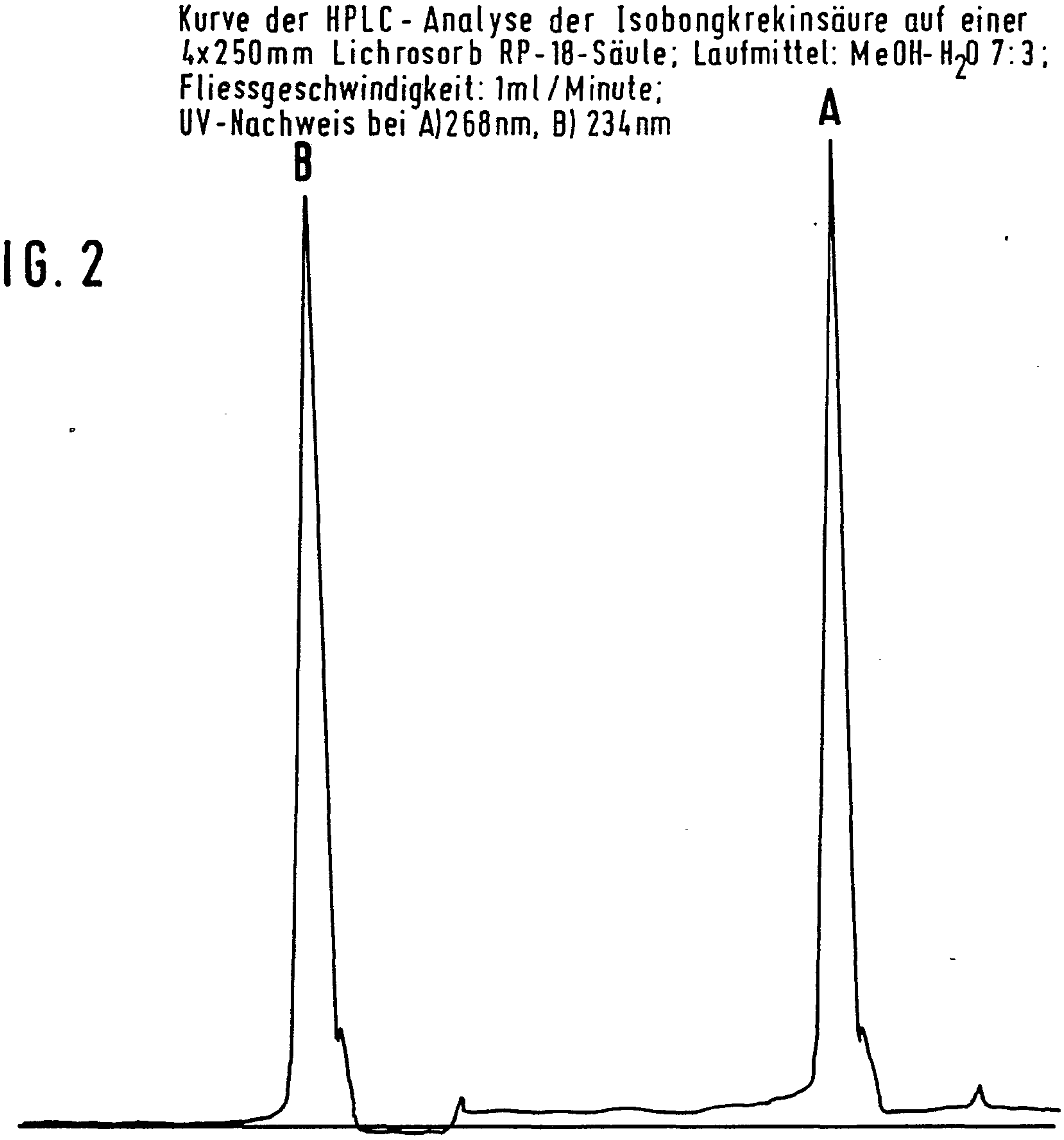

UV-Spektrum der Isobongkrekinsäure (Ia) in MeOH

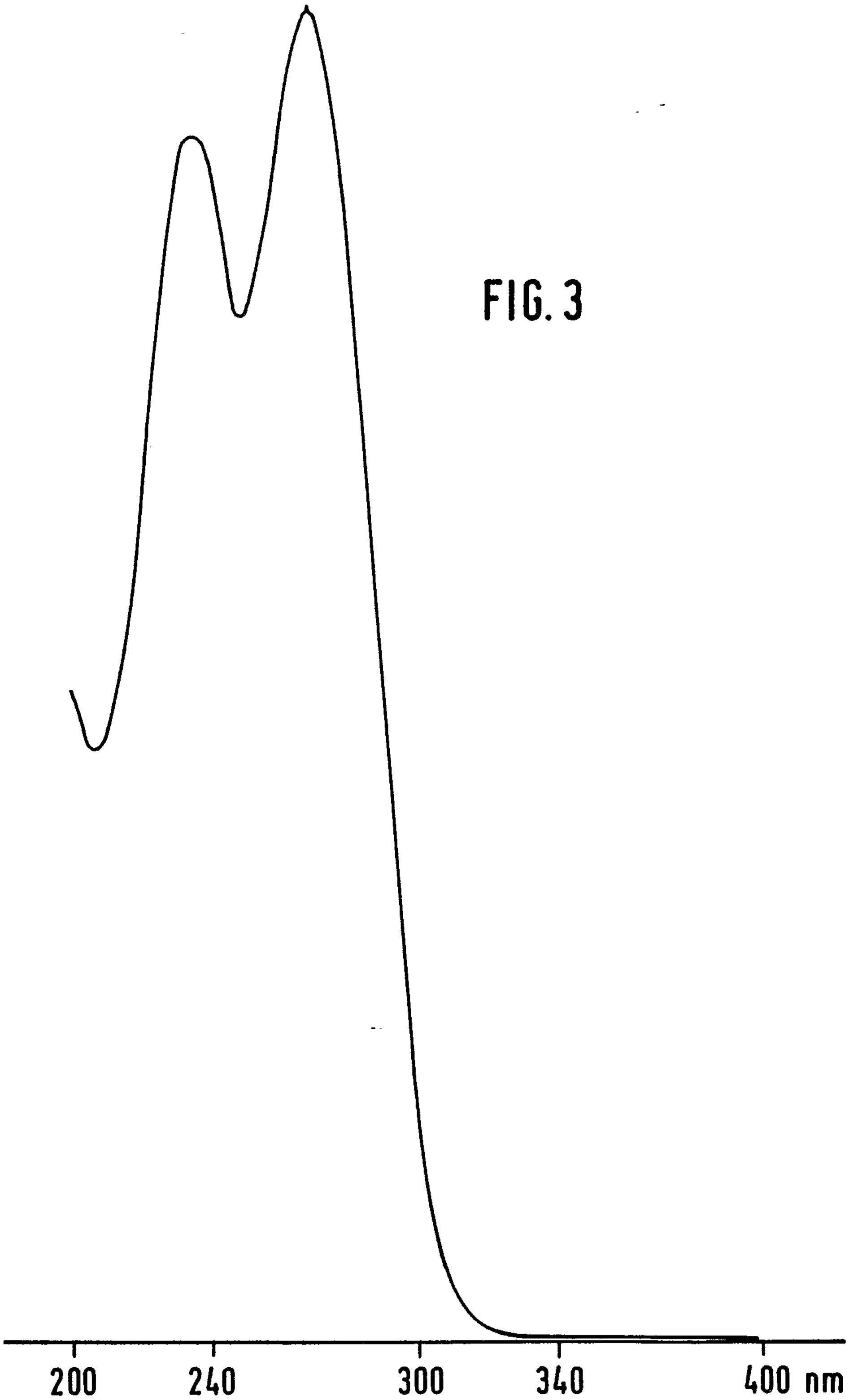


FIG. 3

UV-Spektrum des Isobongkrekinsäuretrimethylesters
(Ib) in MeOH

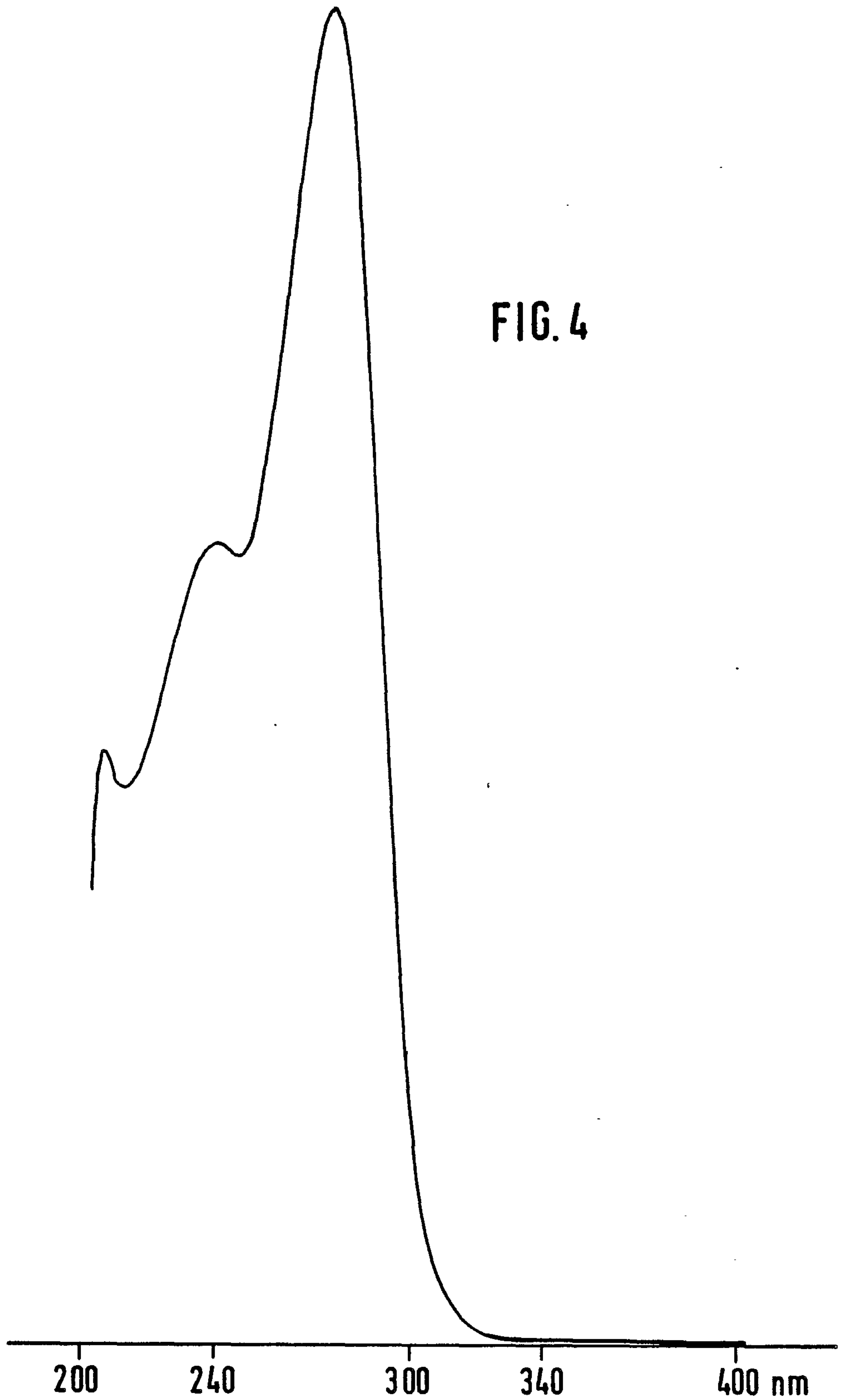


FIG. 4

FIG.5

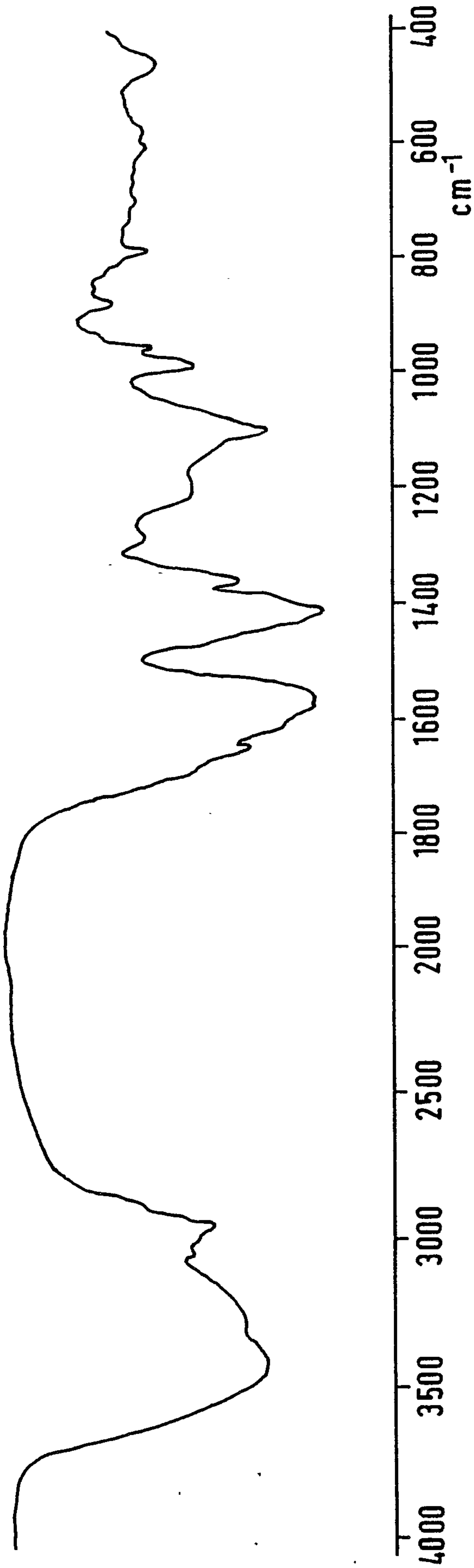


IR-Spektrum der Isobongkrekinsäure (Ia) (in KBr)

FIG. 6

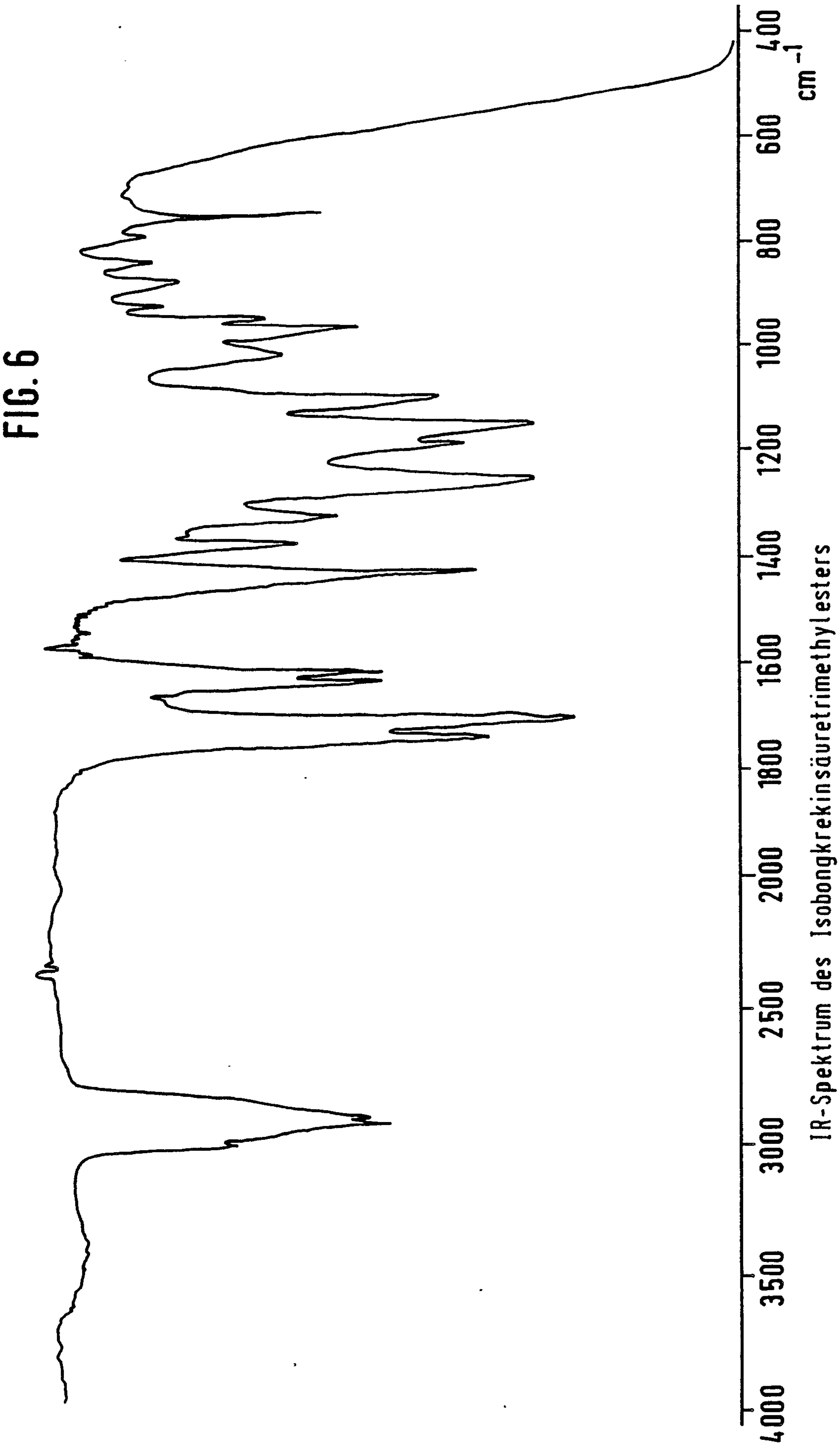


IR-Spektrum des Isobongkrekinsäuretrimethylesters
(Ib) (unverdünnt)

FIG.7

270 MHz $^{1}H$-NMR-Spektrum der Isobongkrekinsäure (Ia) in $D_2O$

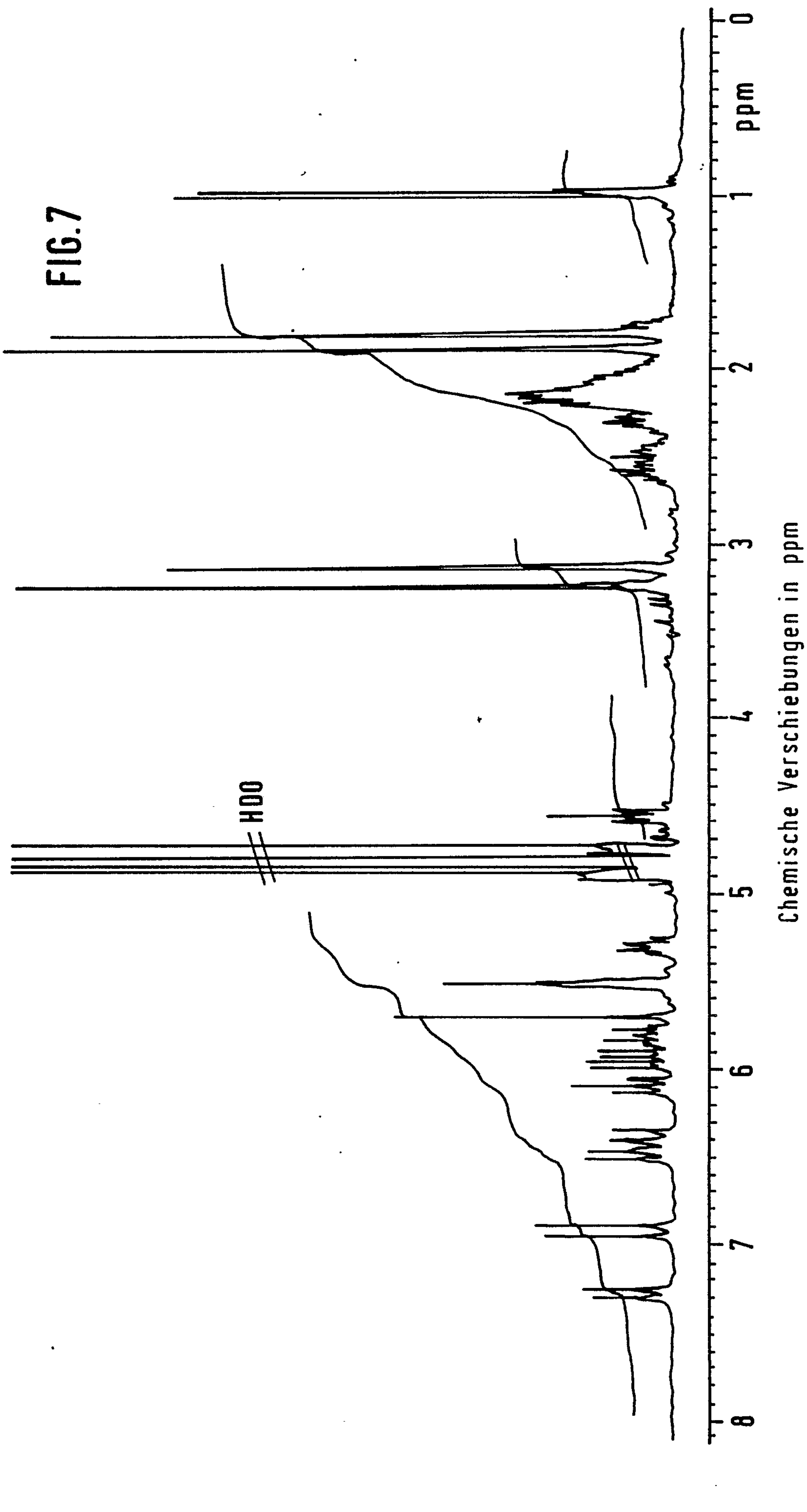

270 MHz $^1$H-NMR-Spektrum des Isobongkrekinsäuretrimethylesters (Ib) in $CDCl_3$

FIG.8

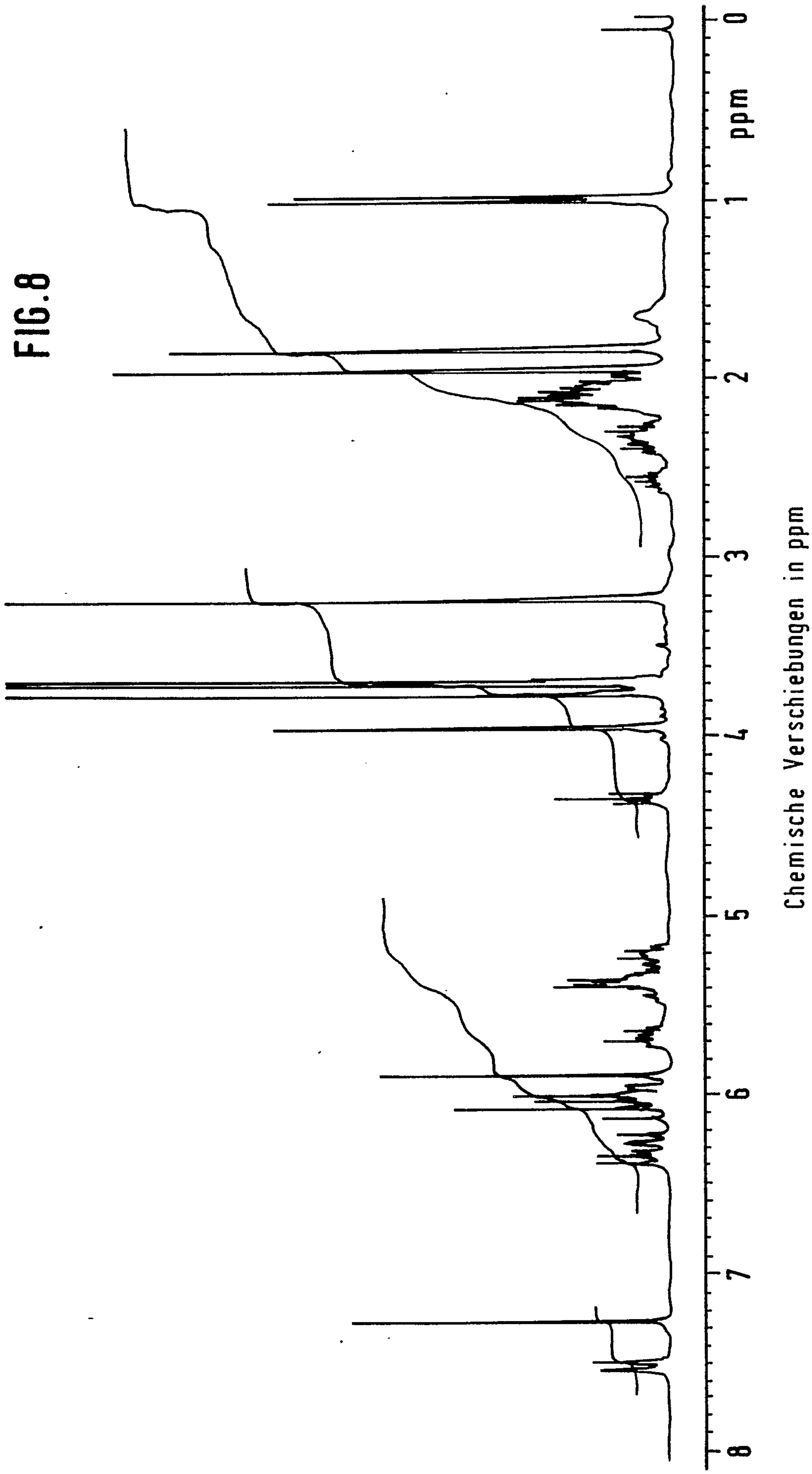

FIG. 9

67,3 MHz $^{13}C$-NMR-Spektrum der Isobongkrekinsäure (I a) in $D_2O$

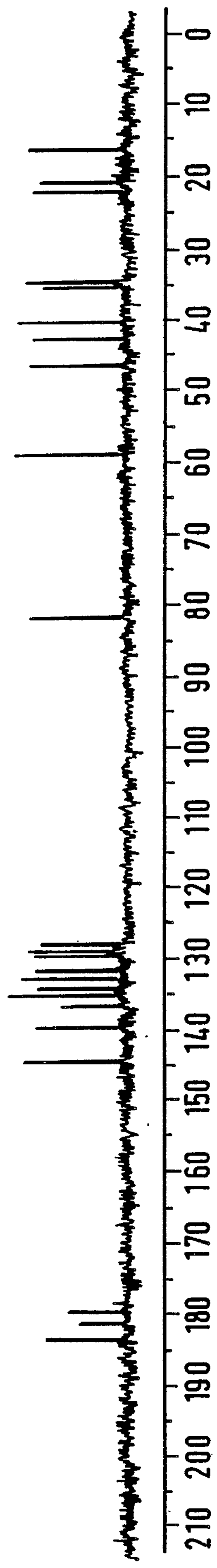


Chemische Verschiebungen in ppm

22,5 MHz $^{13}C$-NMR-Spektrum des Isobongkrekinsäuretrimethylesters (Ib) in $CDCl_3$

FIG. 10